# EUROPEAN PATENT SPECIFICATION

(11) **EP 2 482 262 B1**
(45) Date of publication and mention of the grant of the patent: **06.09.2017**
(21) Application number: 12152848.3
(22) Date of filing: 27.01.2012
(51) Int. Cl.: G08B 15/02, G01N 33/58, C07K 17/00, G09F 3/00, C09D 189/00, B41M 3/00, G01N 33/543

(54) **Marking composition**
Markierungszusammensetzungen
Compositions de marquage

(30) Priority: 27.01.2011 GB 201101411
(43) Date of publication of application: 01.08.2012
(73) Proprietor: APDN (B.V.I.) Inc., Tortola 1110 (VG)
(72) Inventor: Fox, John, Stourbridge, West Midlands DY8 0RX (GB); Taylor, Christopher, Rugeley, Staffordshire WS15 1UZ (GB)
(74) Representative: Wynne-Jones, Lainé and James LLP

(56) References cited:
- WO-A1-95/02702
- WO-A1-95/28640
- GB-A- 2 380 998
- US-A- 4 359 353
- US-A1- 2002 021 003

## Description

This invention relates to a marking composition for uniquely marking a target for identification purposes, a method of manufacturing same, a particulate material, a method of manufacturing said particulate material, and a pressurised container which contains certain marking compositions of the type described herein.

It is known to mark articles with marking compositions which provide unique identifiers which thereby permit the article to be uniquely identified. Various methods and materials for this purpose are described, for example, in GB2319337, GB2380998 and WO2008/007060. In some applications, it is desirable that the marking composition is transferrable onto a person who comes into contact with the article. In this way, it is possible to investigate or identify suspected perpetrators of a theft or an attempted theft of an article. In other applications, a permanent marking composition is provided which is permanently adhered or otherwise attached to an article. By "permanent" it is meant that the marking composition is not easily removed by mechanical contact such as rubbing or wiping, and preferably that the marking composition is not easily removed by washing with water. Marking compositions of this type are useful for authenticating articles such as articles of business or art and various official documents. These kinds of marking compositions also have potential utility in marking a person, such as an assailant or a perpetrator of another crime.

U.S. Patent No. 4,359,353 describes a method of tagging a substance to allow subsequent identification thereof. A polypeptide is incorporated into the substance as a tagging material which can be used to indicate the degree of an oil spill spread.
International Patent Application No. PCT/GB94/01506 describes a security device using nucleic acids attached to beads.

WO95/28640 discloses encoded combinational chemical libraries of compounds such as peptides wherein at each stage of the synthesis a support such as a particle is uniquely tagged to identify the synthesis of the compound on the support.

GB2380998 discloses the use of D-amino peptides for security tagging.

It is well known to provide marking compositions which utilise DNA as a component which provides a unique marking capability. WO2008/007060 is an example of such a prior art system which utilises DNA. However, the present inventors have identified that it would be desirable to utilise peptides as the basis of a unique marking system, because DNA has proven to be more delicate than peptides in real-world usage conditions. In contrast, peptides tend to be less susceptible to degradation and strand breakage than DNA. GB2380998 is an example of a proposed marking system which utilises peptides. However, the present inventors have realised that there are problems associated with the use of peptides in at least some possible applications.

The present invention, in at least some of its embodiments, addresses the above described desires and problems, and provides a peptide-based marking composition which can be convenient to manufacture and can provide efficacious usage properties.

According to a first aspect of the invention there is provided a marking composition for uniquely marking a target for identification purposes which includes a particulate material and at least one substance for attaching the particulate material to the target, in which:
the particulate material includes a plurality of substrate particles each having a peptide possessing a characteristic sequence which is directly bound thereto by a covalent bond wherein each peptide has one or more protecting groups present; and the substance for attaching the particulate material to the target is a matrix material wherein the matrix material is a grease, a grease-like substance, a lacquer or an adhesive.

For the avoidance of doubt, the term "target" is understood herein to refer to objects, articles and also to living beings, typically to humans but, at least in principle, also to animals.

"Free" peptides have limited solubility, and this hinders the incorporation of "free" peptides into some marking compositions. By binding peptides to substrate particles in particulate material, it is possible to conveniently incorporate peptides in a range of marking compositions. Furthermore, the peptides are physically located at discrete and readily identifiable locations, which can assist in the subsequent detection process.

In preferred embodiments, the plurality of substrate particles additionally each have a fluorescent identifier which is directly bound thereto by a covalent bond. The provision of a fluorescent identifier on substrate particles enables the presence of the marking composition to be conveniently identified. Furthermore, this can assist in the detection of the substrate particles themselves.

The marking composition may include a fluorescent identifier which is not bound to the substrate particles.

Advantageously, the substrate particles are beads. In certain embodiments, the substrate particles are formed from a polystyrene. For example, the substrate particles may be Merrifield peptide synthesis resin beads.

The substrate particles may be formed from other polymeric materials, or from non-polymeric materials. Generally, what is required is that the surface of the substrate particles can be provided with a reactive moiety which can be used to directly bind to the peptide (and if required, to the fluorescent identifier). The peptides and, preferably, the fluorescent identifier may be directly bound to the substrate particles by, independently, a peptide, sulfonamide or amine bond linkage. Conveniently, substrate particles such as Merrifield peptide synthesis resin beads can be provided with a reactive amine functionality.

The substrate particles can be of any convenient size. Preferably, the size of the substrate particles is in the range of 20-1000µm. However, substrate articles of a smaller or greater size could be used as desired.

Advantageously, the characteristic sequence of the peptide consists of L-amino acids. The use of L-amino acids is more cost effective than D-amino acids, and is in contrast to the teaching of GB2380998.

Advantageously, the characteristic sequence of the peptide consists of between 5 and 10 amino acids. Surprisingly, it has been found that a relatively low number of amino acids can be sufficient for the purposes of providing unique identification. However, the use of longer sequences of amino acids is within the scope of the invention.

Conveniently, one or more unnatural amino acids may be incorporated into the sequence so as to clearly differentiate the peptide sequence from any naturally occurring peptides.

The peptide has one or more protecting groups present. Advantageously, a Fmoc protecting group (fluorenylmethyloxycarbonyl) is present, although other protecting methodologies might be employed. It is usual in peptide synthesis to utilise protecting groups such as Fmoc groups. However, once synthesised, it is standard practice to remove all of the Fmoc protecting groups. The present inventors have realised that it can be advantageous to retain the final Fmoc protecting groups in place in marking applications, because the resulting peptides are more robust, particularly with respect to enzymatic degradation. Other protecting groups may be utilised, such as to protect amino acid side chains. Protecting groups such as butyl moieties may be used for this purpose. Again, the present inventors have realised that it can be advantageous to retain protecting groups such as these in place after the peptide synthesis is complete.

Typically, the fluorescent identifier is a UV dye, i.e., a dye which fluoresces after exposure to UV radiation. An example of a suitable dye is Dansyl-chloride, although other candidates would suggest themselves to the skilled reader.

The matrix material may provide permanent attachment of the particulate material to the target. In these embodiments, the matrix material may be a grease or a grease-like substance, such as polybutene. Alternatively, the matrix material may provide impermanent attachment of the particulate material to the target so as to permit transfer of marking material on to a person coming into contact with the target. In these embodiments, the matrix material may be a lacquer, such as an acrylic lacquer, or an adhesive.

In a preferred embodiment, the marking composition in the form of an aerosol or in a form suitable for use as an aerosol. In these embodiments, the marking composition may include a propellant. Other suitable forms include sprays, gels and liquids such as paints and inks. Any of these forms can be provided for permanent attachment or impermanent attachment of the particulate material to the target as described above.

Advantageously, the particulate material can be present in relatively low amounts, thereby making the invention economically attractive. Typically, the particular material is present in an amount less than 10% by weight, preferably in the range 0.001 to 5% by weight, more preferably in the range 0.005 to 2.5% by weight, and most preferably about 1 % by weight.

Analysis of the peptide sequence can be performed by any suitable method. The Edman N-terminal sequencing method is preferred as it provides unambiguous sequence results.

According to a second aspect of the invention there is provided a method of manufacturing a marking composition for uniquely marking a target for identification including the steps of:
providing a particulate material which includes a plurality of substrate particles each having a peptide possessing a characteristic sequence which is directly bound thereto by a covalent bond wherein each peptide has one or more protecting groups present;
providing at least one substance for attaching the particulate material to the target; and
incorporating said particulate material and said at least one substance into a marking composition;
in which the substance for attaching the particulate material to the target is a matrix material wherein the matrix material is a grease, a grease-like substance, a lacquer or an adhesive.

According to a third aspect of the invention there is provided a pressurised container for dispersing an aerosol and containing a marking composition as described in the first aspect of the invention in the form of an aerosol or in a form suitable for use as an aerosol. A pressurised container may be pressurised to a desired pressure with the marking composition. Pressures of 60 psi or above may be utilised although the use of lower pressures within the scope of the invention.

Whilst the invention has been described above, it extends to any inventive combination of the features set out above, or in the following description or claims. For example, any feature described in relation to one aspect of the invention can form part of another aspect of the invention.

The invention may be performed in various ways and, by way of example only, embodiments thereof will now be described.

### Attachment of Fluorescent Identifier to Substrate Particles

Suitable substrate particles can be manufactured or obtained from suitable sources. In preferred embodiments, amino methyl polystyrene beads are used which can be purchased from a standard supplier, e.g., Merrifield peptide synthesis resin beads of 100-200µm mesh size can be used, and purchased from various commercial sources such as ChemPep, Inc., 3460 Fairlane Farms Road, Suite 10 Wellington, FL 33414, USA. Other, equivalent resins may be used as would be appreciated by the skilled reader. Beads of this type are based on cross-linked polystyrene, and have an amine functionality as a reactive group. The amine functionality can be reacted with a fluorescent identifier such as a fluorescent dye. For example, Dansyl Chloride (Sigma Aldrich, Gillingham SP8 4XT, UK) was reacted with the beads in 0.5 molar ratio in order to produce beads having the UV dye covalently bound thereto, with approximately 50% of the amine functionality on the resin beads left available for further reaction. In other examples, the polystyrene resin beads were 25% dansylated by performing a reaction with a 0.25 molar ratio of dansyl chloride with respect to the amino methyl groups on the polystyrene resin beads. It is possible to provide polystyrene beads having less than 25% dansylation, or greater than 50% dansylation as appropriate to the end application. Dansylation in the range 5-50% is preferred. In this way, beads were provided which are visible under UV light. These beads were then used as supports for the synthesis of synthetic peptides.

### Peptide Synthesis

Peptide synthesis was performed on the dansylated fluorescent beads using the available amine functionality. A library of random, short peptide sequences was generated by custom written software, and was then transferred onto a database which was rationalised so that each sequence was unique. Methodologies for generating a library of random, short peptide sequences is well known to the skilled reader. A batch of resin beads can then be produced, wherein each bead in the batch has attached thereto peptides of a given unique sequence which is obtained from the database. The synthesis of the peptides can be performed using conventional peptide synthesis chemistry. Such conventional peptide synthesis chemistry is well known to the skilled reader, but additionally we refer to "Fmoc Solid Phase Peptide Synthesis. A Practical Approach." , WC Chan and PD White (Eds), Oxford University Press, 2000, and "Peptide Synthesis and Applications", J Howl (Ed), Humana Press, 2005.

In these examples, Fmoc synthesis with butyl protection of amino acid side chains was performed in order to make the peptides from the C-terminus to N-terminus. At each stage, the Fmoc group was removed using a solution of 20% piperidine in dimethyl formamide. However, the final Fmoc group was left in place, together with the butyl protecting groups. This increases the robustness of the peptides, particularly with respect to enzymatic degradation. Coupling reactions were performed using HCTU(2-(6-chloro-1-H-benzotriazole-1-yl)-1,1,3,3-tetramethylaminium hexafluorophosphate). Other activators may be used, as is well known to the skilled reader. Examples of amino acids which can be used in producing the peptide sequences are as follows: Fmoc Alanine, Fmoc Aspartic
Acid (t.butyl ester), Fmoc Glutamic Acid (t butyl ester), Fmoc Glycine, Fmoc Isoleucine, Fmoc Leucine, Fmoc Lysine (tBoc), Fmoc Methionine, Fmoc Phenylalanine, Fmoc Proline, Fmoc Serine (O tButyl), Fmoc Threonine (O tButyl), Fmoc Tyrosine (O tButyl Ether), and Fmoc Valine. Other amino acids might be utilised, for example Cysteine, Tryptophan, Arginine, Histidine, Asparagine and Glutamine can be used, but are generally less preferred on economic grounds. Additionally, it can be advantageous to include at least one unnatural amino acid in the sequence such as Fmoc amino butyric acid or Fmoc nor Leucine. A wide range of other unnatural amino acids might be used, and other candidates would suggest themselves to the skilled reader. The purpose of using one or more unnatural amino acids is to eliminate any possibility that a detected peptide sequence might have originated from a natural source. Typically, it is quite sufficient to incorporate a single unnatural amino acid into the peptide sequence. It is preferred to use L-amino acids in the synthesis on economic grounds. All of the amino acids can be obtained from conventional sources of supply, such as NovaBiochem, Merck Chemicals Limited, Nottingham NG9 2JR, UK.

In this way, a particulate material can be produced which includes the plurality of substrate particles each of which have a fluorescent identifier and a unique peptide sequence directly bound thereto via a covalent bond. The number ratio of peptide to dye molecules on the substrate particles can be 75: 25, 50:50, or any other desired ratio. The substrate material produced in this way can be incorporated into various marking compositions as described below.

Analysis of the peptide sequence on the substrate particles can be performed by conventional means. Preferably, Edman N-terminal sequencing is employed, using conventional, commercially available sequence instrumentation. It has been found that a single substrate particle can be easily isolated from the particulate material or from marking compositions incorporating same, and thereafter the single beads can be sequenced directly with no ambiguity. Consequently, the fluorescence from the UV dye bound to the beads enables a bead to be easily identified.

### Production of Marking Compositions

Various aerosol marking compositions were prepared as follows using the beads described above which have a UV dye and a characteristic peptide covalently bound thereto (henceforth termed the 'peptide beads'), 'DME' refers to dimethyl ether.

### 1. Grease-based spray with additional dye.

The following aerosol composition was produced:

| Component | Weight Percentage |
|---|---|
| Methylal | 30.7 |
| Polybutene | 29.2 |
| Peptide Beads | 1.0 |
| DME Propellant | 38.7 |
| Aurora FSP-1 Dye | 0.4 |

### 2. Grease-based aerosol without further dye.

An aerosol composition was produced as follows:

| Component | Weight Percentage |
|---|---|
| Methylal | 31.1 |
| Polybutene | 29.2 |
| Peptide Beads | 1.0 |
| DME Propellant | 38.7 |

### 3. Lacquer-based spray with additional dye.

An aerosol composition was produced as follows:

| Component | Weight Percentage |
|---|---|
| Acetone | 26.8 |
| Acrylic Lacquer | 24.9 |
| Peptide Beads | 1.0 |
| Amorphous Silica Matting Agent | 0.5 |
| Butene/Propene Propellant | 46.3 |
| Aurora SSP-1 Dye | 0.5 |

### 4. Lacquer-based spray without additional dye.

An aerosol composition was produced as follows:

| Component | Weight Percentage |
|---|---|
| Acetone | 27.3 |
| Acrylic Lacquer | 24.9 |
| Peptide Beads | 1.0 |
| Amorphous Silica Matting Agent | 0.5 |
| Butene/Propene Propellant | 46.3 |

The aerosol compositions 1 and 3, which contain the additional dye Aurora SSP-1, are suitable for more "overt" applications. Aerosol compositions 2 and 4 are suitable for more "covert" applications. For these applications, it has been found that the peptide beads of the invention provide excellent fluorescence without being blanched by the red fluorescence of the additional, Aurora SSP-1 dye.

The peptide beads may be introduced directly into the aerosol formulation, or may be introduced as a suspension in a liquid carrier such as water. The suspension may have additional components such as sodium benzoate or a small amount of a stain such as erythrosine.

Aerosol-based marking compositions of this type can be used to mark the perpetrator of a crime as well as for marking inanimate objects.

Marking compositions of different types may be provided. In one further example, a liquid marking composition was produced as follows:

| Component | Weight Percentage |
|---|---|
| Butyl Acetate | 59.99 |
| MEK (Butanone) | 40 |
| Peptide Beads | 0.01 |

Compositions of this type having greater amounts of the peptide beads can be produced. The peptide beads are present in a liquid carrier and can be agitated into a suspension using, for example, small ball bearing. The marking composition can be applied to a target area using a small applicator brush. A lacquer, such as an acrylic or water-based lacquer may be added to improve the attachment of the peptide beads to the target to be marked. Compositions of this type are especially useful for the marking of assets.

## Claims

1. A marking composition for uniquely marking a target for identification purposes which includes a particulate material and at least one substance for attaching the particulate material to the target, in which:
the particulate material includes a plurality of substrate particles each having a peptide possessing a characteristic sequence which is directly bound thereto by a covalent bond wherein each peptide has one or more protecting groups present; and
the substance for attaching the particulate material to the target is a matrix material wherein the matrix material is a grease, a grease-like substance, a lacquer or an adhesive.

2. A marking composition according to Claim 1 in which the plurality of substrate particles additionally each have directly bound thereto a fluorescent identifier which is directly bound thereto by a covalent bond.

3. A marking composition according to Claim 1 or Claim 2 in which the substrate particles are beads.

4. A marking composition according to any one of Claims 1 to 3 in which the substrate particles are formed from a polystyrene.

5. A marking composition according to any one of Claims 1 to 4 in which the peptide and, preferably, the fluorescent identifier are directly bound to the substrate particles by, independently, a peptide, sulphonamide or amine bond linkage.

6. A marking composition according to any previous claim in which the size of the substrate particles is in the range 20 to 1000µm.

7. A marking composition according to any previous claim in which the characteristic sequence of the peptide consists of L-amino acids.

8. A marking composition according to any previous claim in which the characteristic sequence of the peptide consists of between five and ten amino acids.

9. A marking composition according to any previous claim in which a Fmoc protecting group is present.

10. A marking composition according to any previous claim in the form of an aerosol or in a form suitable for use as an aerosol.

11. A marking composition according to any previous claim in which the particulate material is present in an amount less than 10% by weight, preferably in the range 0.001 to 5% by weight, more preferably in the range 0.005 to 2.5% by weight, most preferably about 1 % by weight.

12. A method of manufacturing a marking composition for uniquely marking a target for identification including the steps of:
providing a particulate material which includes a plurality of substrate particles each having a peptide possessing a characteristic sequence which is directly bound thereto by a covalent bond, wherein each peptide has one or more protecting groups present;
providing at least one substance for attaching the particulate material to the target; and
incorporating said particulate material and said at least one substance into a marking composition;
in which the substance for attaching the particulate material to the target is a matrix material wherein the matrix material is a grease, a grease-like substance, a lacquer or an adhesive.

13. A pressurised container for dispersing an aerosol and containing a marking composition according to claim 1.

## Patentansprüche

1. Markierungszusammensetzung zum eindeutigen Markieren eine Ziels zu Identifizierungszwecken, die ein teilchenförmiges Material und mindestens eine Substanz zum Binden des teilchenförmigen Materials an das Ziel umfasst, wobei:
das teilchenförmige Material eine Mehrzahl von Substratteilchen umfasst, die jeweils ein Peptid aufweisen, das eine charakteristische Sequenz aufweist und das durch eine kovalente Bindung direkt daran gebunden ist, wobei an jedem Peptid eine oder mehrere Schutzgruppe(n) vorliegt oder vorliegen; und
die Substanz zum Binden des teilchenförmigen Materials an das Ziel ein Matrixmaterial ist, wobei das Matrixmaterial ein Fett, eine fettartige Substanz, ein Lack oder ein Haftmittel ist.

2. Markierungszusammensetzung nach Anspruch 1, bei der an die Mehrzahl von Substratteilchen zusätzlich jeweils ein fluoreszierendes Identifiziermittel direkt gebunden ist, das durch eine kovalente Bindung direkt daran gebunden ist.

3. Markierungszusammensetzung nach Anspruch 1 oder Anspruch 2, bei der die Substratteilchen Kügelchen sind.

4. Markierungszusammensetzung nach einem der Ansprüche 1 bis 3, bei der die Substratteilchen aus einem Polystyrol ausgebildet sind.

5. Markierungszusammensetzung nach einem der Ansprüche 1 bis 4, bei der das Peptid und vorzugsweise das fluoreszierende Identifiziermittel unabhängig durch eine Peptid-, Sulfonamid- oder Aminbindungsverknüpfung direkt an die Substratteilchen gebunden sind.

6. Markierungszusammensetzung nach einem der vorhergehenden Ansprüche, bei der die Größe der Substratteilchen im Bereich von 20 bis 1000 µm liegt.

7. Markierungszusammensetzung nach einem der vorhergehenden Ansprüche, bei der die charakteristische Sequenz des Peptids aus L-Aminosäuren besteht.

8. Markierungszusammensetzung nach einem der vorhergehenden Ansprüche, bei der die charakteristische Sequenz des Peptids aus zwischen fünf und zehn Aminosäuren besteht.

9. Markierungszusammensetzung nach einem der vorhergehenden Ansprüche, bei der eine Fmoc-Schutzgruppe vorliegt.

10. Markierungszusammensetzung nach einem der vorhergehenden Ansprüche in der Form eines Aerosols oder in einer Form, die zur Verwendung als Aerosol geeignet ist.

11. Markierungszusammensetzung nach einem der vorhergehenden Ansprüche, bei der das teilchenförmige Material in einer Menge von weniger als 10 Gew.-%, vorzugsweise im Bereich von 0,001 bis 5 Gew.-%, mehr bevorzugt im Bereich von 0,005 bis 2,5 Gew.-%, insbesondere etwa 1 Gew.-%, vorliegt.

12. Verfahren zur Herstellung einer Markierungszusammensetzung zum eindeutigen Markieren eine Ziels zu Identifizierungszwecken, umfassend die Schritte:
Bereitstellen eines teilchenförmigen Materials, das eine Mehrzahl von Substratteilchen umfasst, die jeweils ein Peptid aufweisen, das eine charakteristische Sequenz aufweist und das durch eine kovalente Bindung direkt daran gebunden ist, wobei an jedem Peptid eine oder mehrere Schutzgruppe(n) vorliegt oder vorliegen;
Bereitstellen mindestens einer Substanz zum Binden des teilchenförmigen Materials an das Ziel; und
Einbringen des teilchenförmigen Materials und der mindestens einen Substanz in eine Markierungszusammensetzung;
wobei die Substanz zum Binden des teilchenförmigen Materials an das Ziel ein Matrixmaterial ist, wobei das Matrixmaterial ein Fett, eine fettartige Substanz, ein Lack oder ein Haftmittel ist.

13. Mit Druck beaufschlagter Behälter zum Ausbringen eines Aerosols und der eine Markierungszusammensetzung nach Anspruch 1 enthält.

## Revendications

1. Composition de marquage pour marquer de manière unique une cible à des fins d'identification qui comprend une matière particulaire et au moins une substance pour attacher la matière particulaire à la cible, dans laquelle :
la matière particulaire comprend une pluralité de particules de substrat ayant chacune un peptide possédant une séquence caractéristique qui est directement liée à celui-ci par une liaison covalente, chaque peptide ayant un ou plusieurs groupes protecteurs présents ; et
la substance pour attacher la matière particulaire à la cible est une matière de matrice, la matière de matrice étant une graisse, une substance analogue à une graisse, une laque ou un adhésif.

2. Composition de marquage selon la revendication 1, dans laquelle les particules de substrat ont chacune additionnellement directement lié à celles-ci un identifiant fluorescent qui est directement lié à celles-ci par une liaison covalente.

3. Composition de marquage selon la revendication 1 ou la revendication 2, dans laquelle les particules de substrat sont des perles.

4. Composition de marquage selon l'une quelconque des revendications 1 à 3, dans laquelle les particules de substrat sont formées à partir d'un polystyrène.

5. Composition de marquage selon l'une quelconque des revendications 1 à 4, dans laquelle le peptide et, de préférence, l'identifiant fluorescent sont directement liés aux particules de substrat par, indépendamment, une liaison peptide, sulfonamide ou amine.

6. Composition de marquage selon l'une quelconque des revendications précédentes, dans laquelle la dimension des particules de substrat se situe dans la plage de 20 à 1000 µm.

7. Composition de marquage selon l'une quelconque des revendications précédentes, dans laquelle la séquence caractéristique du peptide consiste en L-amino acides.

8. Composition de marquage selon l'une quelconque des revendications précédentes, dans laquelle la séquence caractéristique du peptide consiste entre cinq et dix acides aminés.

9. Composition de marquage selon l'une quelconque des revendications précédentes, dans laquelle un groupe protecteur Fmoc est présent.

10. Composition de marquage selon l'une quelconque des revendications précédentes sous la forme d'un aérosol ou sous une forme appropriée pour une utilisation comme aérosol.

11. Composition de marquage selon l'une quelconque des revendications précédentes, dans laquelle la matière particulaire est présente dans une quantité inférieure à 10 % en poids, de préférence dans la plage de 0,001 à 5 % en poids, de façon davantage préférée dans la plage de 0,005 à 2,5 % en poids, de la façon que l'on préfère le plus d'environ 1 % en poids.

12. Procédé de fabrication d'une composition de marquage pour marquer de manière unique une cible pour l'identification comprenant les étapes de :
fournir une matière particulaire qui comprend une pluralité de particules de substrat ayant chacune un peptide possédant une séquence caractéristique qui est directement liée à celui-ci par une liaison covalente, chaque peptide ayant un ou plusieurs groupes protecteurs présents ;
fournir au moins une substance pour attacher la matière particulaire à la cible ; et
incorporer ladite matière particulaire et ladite au moins une substance dans une composition de marquage ;
dans lequel la substance pour attacher la matière particulaire à la cible est une matière de matrice, la matière de matrice étant une graisse, une substance analogue à une graisse, une laque ou un adhésif.

13. Conteneur pressurisé pour disperser un aérosol et contenant une composition de marquage selon la revendication 1.
